# DEMANDE DE BREVET EUROPEEN

(11) **EP 0 644 424 A1**
(43) Date de publication de la demande: **22.03.1995**
(21) Numéro de dépôt: 94114426.3
(22) Date de dépôt: 14.09.1994
(51) Int. Cl.: G01N 33/487, G01N 33/49

(54) **Dispositif de mesure pour capteurs multizones amovibles**

(30) Priorité: 21.09.1993 FR 9311317
(71) Demandeur: ASULAB S.A., CH-2501 Bienne (CH)
(72) Inventeur: Hoffmann, Eric, CH-2563 Ipsach (CH); Desarzens, Pierre, CH-2503 Bienne (CH)
(74) Mandataire: Patry, Didier Marcel Pierre

(57) **Abrégé**

La présente invention concerne un dispositif de mesure (102), notamment pour la mesure du taux de glucose dans le sang, destiné à être associé à un capteur amovible (108) comprenant une pluralité de zones actives (144).

En particulier, l'invention concerne un connecteur mobile (150) assurant la connection électrique entre le capteur amovible (108) et un circuit électronique de mesure solidaire du boîtier (104) de ce dispositif de mesure. Cette connection électrique est réalisée au moyen de contacts électriques glissants.

## Description

La présente invention concerne un dispositif de mesure destiné à être associé à un capteur amovible comportant une pluralité de zones actives à usage unique. Un tel capteur est nommé par la suite "capteur multizone".

Plus particulièrement, la présente invention concerne un connecteur mobile servant à relier électriquement un circuit électrique ou électronique de mesure, agencé dans un boîtier du dispositif de mesure, à un capteur multizone amovible servant à effectuer une pluralité de mesures, notamment de mesures électrochimiques.

Aux figures 1 et 2 est représenté un dispositif de mesure électrochimique tel que décrit dans la demande de brevet FR 92 01331.

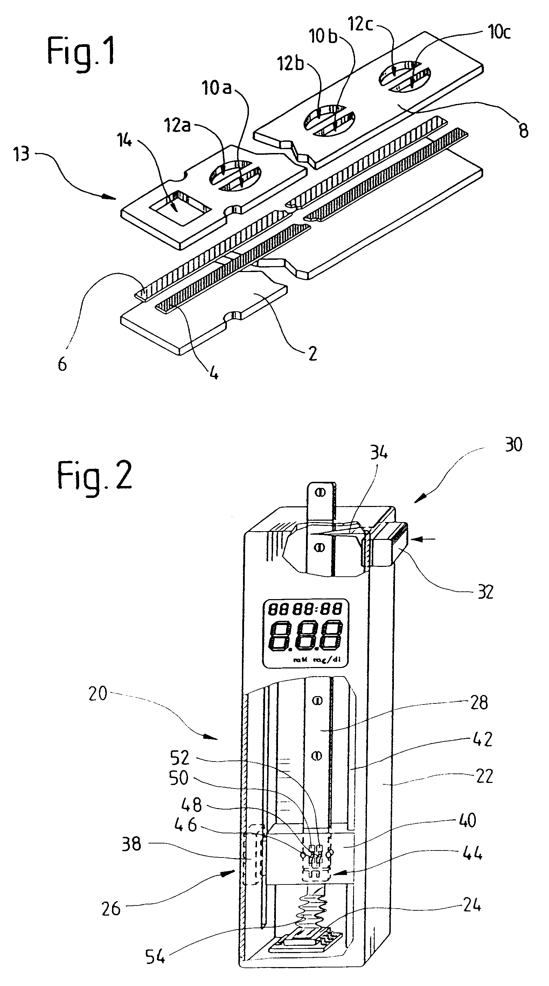

Sur la figure 1 est représenté partiellement et en perspective un éclaté d'un capteur multizone comportant un substrat isolant 2 à la surface duquel sont prévus deux conducteurs 4 et 6 servant à conduire un courant électrique de mesure. Sur ces conducteurs 4 et 6 est prévu un revêtement 8 également isolant.

Le revêtement 8 comporte une première série d'ouvertures 10a, 10b, 10c au moins partiellement superposées au premier conducteur 4 et une deuxième série d'ouvertures 12a, 12b, 12c superposées au moins partiellement au deuxième conducteur 6. A une extrémité 13 du revêtement 8 est prévue au moins une ouverture 14 servant à dégager une plage de contact électrique sur chacun des deux conducteurs 4 et 6.

On notera que les conducteurs 4 et 6 sont électriquement isolés l'un de l'autre et que la première série d'ouvertures 10a, 10b, 10c n'est pas superposée au deuxième conducteur 6. De même, la deuxième série d'ouvertures 12a, 12b, 12c n'est pas superposée au premier conducteur 4. Chaque paire d'ouvertures correspondantes 10a et 12a, respectivement 10b et 12b, 10c et 12c définit une zone active du capteur multizone. Le fonctionnement électrochimique d'un tel capteur multizone est sensiblement identique à celui d'un capteur monozone tel que décrit par exemple dans la demande de brevet WO 92/14836.

La substance à analyser, par exemple du sang dans une application possible à la mesure du taux de glucose dans le sang, est apportée dans une des zones actives du capteur multizone. Pour effectuer une mesure électrochimique valable, il est nécessaire que seulement une des zones actives du capteur multizone soit recouverte par la substance à analyser, les zones actives restantes devant nécessairement être non-utilisées.

Sur la figure 2 est représenté un dispositif de mesure électrochimique, désigné par la référence générale 20, destiné à recevoir un capteur multizone amovible du type de celui décrit à la figure 1.

Le dispositif de mesure électrochimique 20 comporte un boîtier 22 à l'intérieur duquel est prévue une électronique de mesure 24. En outre, ce dispositif de mesure électrochimique 20 comprend un dispositif d'avance 26 servant à déplacer le capteur multizone 28 introduit à l'intérieur du boîtier 22.

Afin de couper, après utilisation d'une zone active pour effectuer une mesure électrochimique, le capteur multizone 28 pour séparer la partie de ce capteur multizone 28 comportant cette zone active utilisée du reste de celui-ci, il est prévu un dispositif de coupe 30.

Le dispositif de coupe 30 comprend un poussoir 32 et une lame 34 solidaire de ce poussoir 32. Le dispositif de coupe 30 est agencé de manière que le poussoir 32 est susceptible d'effectuer une course, selon une direction transversale au capteur multizone 28, suffisamment longue pour permettre à la lame 34 de traverser entièrement ce capteur multizone 28.

Le dispositif d'avance 26 comporte un poussoir extérieur 38 solidaire d'un chariot mobile 40 auquel est fixé le capteur multizone 28. Ce chariot mobile 40 est susceptible de coulisser le long d'un rail de guidage 42.

En outre, le chariot mobile 40 est relié solidement à un connecteur 44 comprenant deux organes de contact électrique 46 et 48 servant à établir respectivement deux connexions électriques avec deux plages de contact 50 et 52 prévues sur ledit capteur multizone amovible 28.

D'autre part, le connecteur 44 est relié électriquement à l'électronique de mesure 24, solidaire du boîtier 22, au moyen d'un câble multiconducteur souple 54, ce câble 54 étant plat et susceptible d'être plissé en accordéon.

La connexion électrique entre le connecteur 44 et l'électronique de mesure 24 établie par le câble multiconducteur souple 54 présente certains inconvénients. Premièrement, étant donné que le câble multiconducteur souple 54 est soumis alternativement à des contractions et des extensions, il s'ensuit des contraintes mécaniques exercées sur les soudures de connexion entre l'extrémité du câble multiconducteur souple 54 et l'électronique de mesure 24. Ainsi, le dispositif de mesure 20 est susceptible de subir une détérioration relativement rapide.

Deuxièmement, le long parcours effectué par le chariot mobile 40 à l'intérieur du boîtier 20 nécessite un câble multiconducteur souple 54 suffisamment long pour permettre au chariot mobile 40 d'effectuer ce long parcours. De ce fait, il est difficile d'obtenir une contraction correcte du câble multiconducteur souple 54 lorsque le chariot mobile 40 est déplacé en direction de l'électronique de mesure 24. Il a été constaté que le câble 54 a tendance à se développer dans l'espace libre à l'intérieur du boîtier 22 lorsque le chariot mobile 40 est amené dans une position de retrait en direction de l'électronique de mesure 24. Ceci augmente les contraintes mécaniques sur les soudures de connexion entre le câble 54 et l'électronique de mesure 24 et entre ce câble 54 et le connecteur 44.

Le but de la présente invention est de pallier les inconvénients mentionnés ci-dessus en fournissant un dispositif de mesure équipé de moyens de connexion entre un capteur multizone amovible et une électronique de mesure, prévue dans ce dispositif de mesure, fiables et adaptés au caractère mobile du capteur multizone amovible associé à ce dispositif de mesure.

La présente invention a donc pour objet un dispositif de mesure, destiné à être associé à un capteur multizone amovible, comprenant un boîtier, un circuit électrique de mesure agencé dans ce boîtier et un connecteur mobile servant à relier électriquement ce circuit électrique de mesure audit capteur multizone amovible. Le connecteur mobile comprend des premiers moyens de contact électrique destinés à relier électriquement ce connecteur mobile au capteur multizone amovible et des deuxièmes moyens de contact électrique reliés électriquement aux premiers moyens de contact électrique et servant à relier électriquement ce connecteur mobile au circuit électrique de mesure. Ce dispositif de mesure est caractérisé en ce que les deuxièmes moyens de contact électrique sont formés par au moins deux organes de contact électrique et en ce qu'il comprend deux collecteurs fixes ménagés à l'intérieur du boîtier de manière que les deux organes de contact électrique sont respectivement en contact électrique avec les deux collecteurs fixes lorsque ledit connecteur mobile est situé dans une quelconque position de mesure parmi une pluralité de positions de mesure différentes.

Selon un mode de réalisation préféré de l'invention, les deux collecteurs fixes sont formés respectivement par deux pistes métallisées contre lesquelles lesdits deux organes de contact électrique sont respectivement en appui de manière à former deux contacts électriques glissants entre ces deux pistes métallisées et ces deux organes de contact électrique.

Ainsi, la connexion électrique entre le capteur multizone amovible introduit dans le dispositif de mesure et le circuit électrique est assurée sans la présence d'un câble multiconducteur souple. De plus, aucune soudure entre le connecteur mobile et le circuit électrique de mesure n'est nécessaire. La connexion électrique entre ce connecteur mobile et ce circuit électrique est fiable et non sujette à des contraintes mécaniques lors du déplacement du connecteur mobile solidaire dudit capteur multizone amovible, si ce n'est le frottement des deux organes de contact électrique sur les deux collecteurs fixes respectifs.

D'autres caractéristiques et avantages de l'invention seront décrits ci-après à l'aide de la description suivante faite en référence aux dessins annexés, donnés à titre d'exemples nullement limitatifs, dans lesquels :
- la figure 1, déjà décrite, montre en perspective un éclaté d'un capteur multizone amovible;
- la figure 2, déjà décrite, montre schématiquement un dispositif de mesure associé au capteur multizone amovible représenté à la figure 1;
- la figure 3 est une vue schématique partiellement arrachée d'un mode de réalisation d'un dispositif de mesure selon l'invention;
- la figure 4 est une vue partielle agrandie de la figure 3 montrant un connecteur mobile selon l'invention;
- la figure 5 est une vue en coupe selon la ligne de coupe V - V de la figure 4;
- la figure 6 est une vue similaire à la figure 4, mais montrant une variante de réalisation du connecteur mobile;
- la figure 7 est une vue de côté du connecteur mobile représenté à la figure 6.

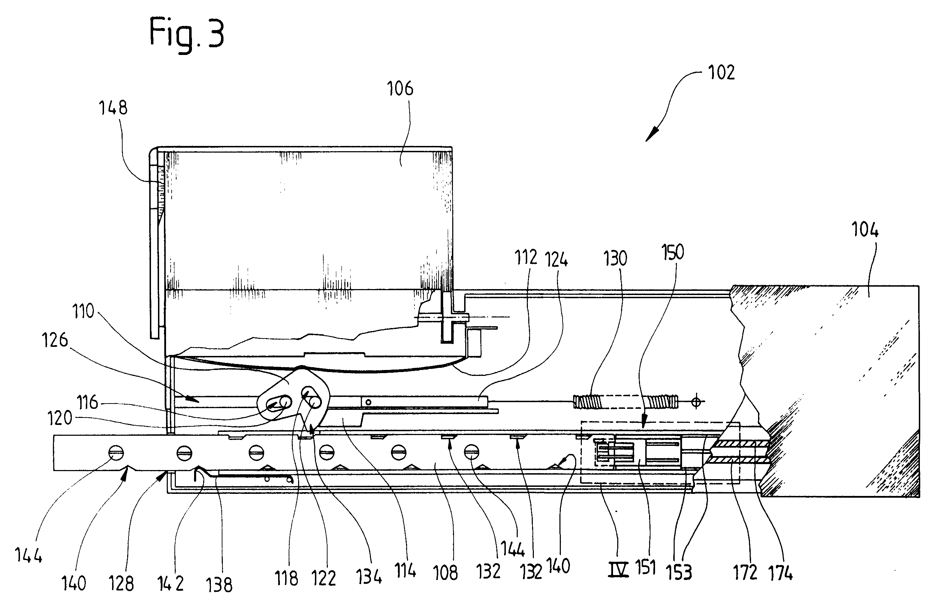

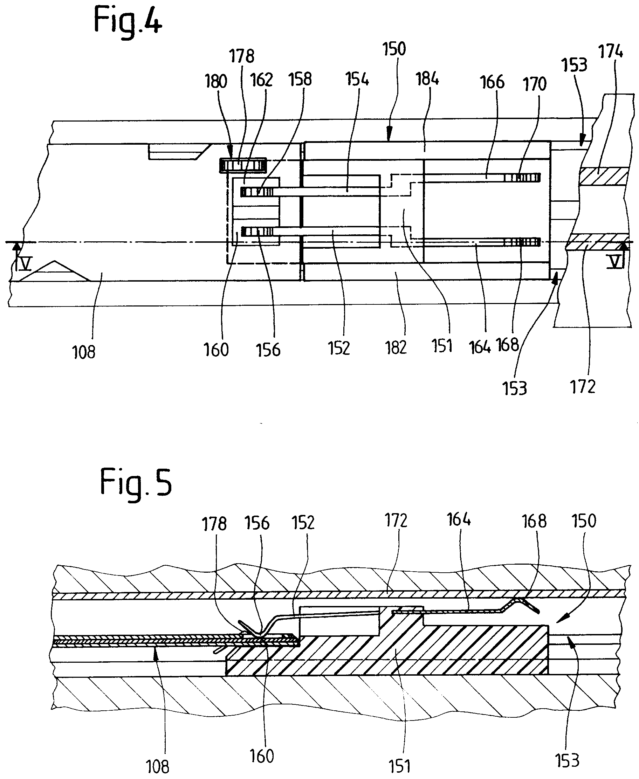

A l'aide des figures 3 à 5, on décrira ci-après un mode de réalisation d'un dispositif de mesure selon l'invention.

Sur la figure 3 est représenté un dispositif de mesure 102 comportant un boîtier 104 dont la partie supérieure à été partiellement arrachée. Le dispositif de mesure 102 est destiné à être associé à un capteur multizone amovible 108. Afin de permettre le déplacement du capteur multizone amovible 108 introduit à l'intérieur du boîtier 104, il est prévu dans ce boîtier 104 un système d'avance comportant une butée mobile 110 associée à une lame élastique 112 et une butée fixe 114 solidaire du boîtier 104.

La butée mobile 110 comporte deux ouvertures oblongues 116 et 118 dans lesquelles sont respectivement placés deux tétons 120 et 122 faisant saillie d'une barrette mobile 124 guidée dans une coulisse 126. La barrette 124 est reliée solidairement à un poussoir (non représenté) permettant de déplacer la butée mobile 110 depuis sa position de repos, dans laquelle elle est représentée sur la figure 3, en direction de l'orifice 128 servant à l'introduction du capteur multizone amovible 108 à l'intérieur du boîtier 104.

Il est également prévu un ressort de rappel 130 relié à une extrémité à la barrette 124 et à l'autre extrémité au boîtier 104. Ce ressort de rappel 130 permet de ramener la butée mobile 110 dans sa position de repos lorsqu'elle a été déplacée par un utilisateur pour faire avancer le capteur multizone amovible 108 à l'intérieur du boîtier 104. Le dispositif d'avance prévu à l'intérieur du boîtier 104 est associé à des crans d'avance 132 disposés le long du capteur multizone amovible 108. La butée mobile 110 est agencée de manière que son extrémité 134 s'emboîte dans un des crans d'avance 132 lorsque la butée mobile est actionnée au moyen du poussoir (non représenté) par un utilisateur.

Il est également prévu dans le boîtier 104 un ressort de positionnement 138 coopérant avec des encoches de positionnement 140 prévues le long du capteur multizone amovible 108. Le ressort de positionnement 138 associé aux encoches de positionnement 140 définit une pluralité de positions de mesure différentes du capteur multizone amovible 108 relativement au boîtier 104.

On notera que les encoches de positionnement 140 sont disposées à intervalles réguliers le long du capteur multizone amovible 108. De même, les crans d'avance 132 sont disposés le long de ce capteur multizone amovible 108 à intervalles réguliers identiques aux intervalles réguliers prévus entre les encoches de positionnement 140.

La position des encoches de positionnement 140 relativement aux crans d'avance 132 est déterminée en fonction de la position du crochet 142 du ressort de positionnement 138 qui assure le maintien dans une quelconque position de mesure du capteur multizone amovible 108 et de la butée fixe 114 qui détermine la position de repos de la butée mobile 110.

Dans le cas où le capteur multizone amovible 108 comporte des zones actives 144 à usage unique, il est nécessaire de couper ce capteur multizone amovible 108 après chaque mesure afin de séparer la zone active utilisée pour cette mesure des autres zones actives non-utilisées. Pour ce faire, il est prévu une lame 148 agencée dans le couvercle 106 du dispositif de mesure 102, cette lame 148 subissant un mouvement de rotation solidaire du couvercle 106. Ainsi, il est prévu qu'un utilisateur actionne le couvercle 106 pour couper le capteur multizone 108 entre deux mesures consécutives.

A l'aide des figures 3 à 5, on décrira ci-après plus particulièrement le dispositif de connexion électrique entre un circuit électrique de mesure ou une électronique de mesure (non représentée), comprise à l'intérieur du boîtier 104, et le capteur multizone amovible 108.

Selon l'invention, le système de connexion électrique entre le capteur multizone amovible 108 et une électronique de mesure solidaire du boîtier 104 comporte un connecteur mobile 150 comprenant un chariot mobile 151 guidé dans une coulisse 153 ménagée à l'intérieur du boîtier 104. Le connecteur mobile 150 comprend en outre des premiers moyens de contact électrique formés par deux organes de contact électrique 152 et 154. Ces deux organes de contact électrique 152 et 154 sont formés respectivement par deux lames métalliques élastiques comportant chacune une extrémité libre recourbée 156, 158 et servent à établir respectivement deux contacts électriques par pression avec deux plages de contact électrique respectives 160, 162 prévues sur une partie terminale du capteur multizone amovible 108.

Le connecteur mobile 150 comporte également des deuxièmes moyens de contact électrique formés dans ce mode de réalisation par deux organes de contact électrique 164 et 166. Les deux organes de contact électrique 152 et 154 appartenant aux premiers moyens de contact électrique sont reliés électriquement et respectivement aux deux organes de contact électrique 164 et 166.

Les deux organes de contact électriques 164 et 166 sont constitués également par deux lames métalliques élastiques comportant chacune une extrémité recourbée 168, 170 formant un crochet et servent à établir respectivement deux contacts électriques par pression avec deux collecteurs fixes respectifs 172, 174. Ces deux collecteurs fixes respectifs 172, 174, constitués dans ce mode de réalisation par deux pistes métallisées continues, forment en association avec les deux organes de contact électrique respectifs 164 et 166 deux contacts électriques glissants assurant ainsi une connexion électrique fiable entre le connecteur mobile 150 relié électriquement au capteur multizone amovible 108 et une électronique de mesure (ou circuit électrique de mesure) non représentée.

Dans le mode de réalisation décrit ici, les deux organes de contact électrique 152 et 154 forment respectivement avec les deux organes de contact électrique 164 et 166, auxquels ils sont respectivement reliés électriquement, deux structures plates fixées solidement au chariot mobile 151 et isolées électriquement l'une de l'autre. On notera aussi que les deux pistes métallisées 172 et 174 sont isolées électriquement l'une de l'autre. Ainsi, le connecteur mobile 150 associé aux deux pistes métallisées 172 et 174 permet d'établir un circuit électrique entre chacune des zones actives 144 du capteur multizone amovible 108 et l'électronique de mesure mentionnée ci-avant.

Afin de fixer solidement, mais de manière réversible, le capteur multizone amovible 108 au connecteur mobile 150, il est prévu un crochet 178 associé à une ouverture 180 prévue dans ce capteur multizone amovible 108. Le crochet 178 est susceptible de subir un déplacement perpendiculairement au plan général du capteur multizone amovible 108 de telle manière à permettre la fixation de ce capteur 108 au connecteur mobile 150 ou sa libération de ce dernier. Tout autre moyen de fixation réversible connu de l'homme du métier et approprié à un tel agencement est naturellement envisageable.

On notera encore que le chariot mobile 151 comporte deux rails de guidage 182 et 184 qui coopèrent avec la coulisse 153 pour guider ce chariot mobile 151 à l'intérieur du boîtier 104 du dispositif de mesure 102.

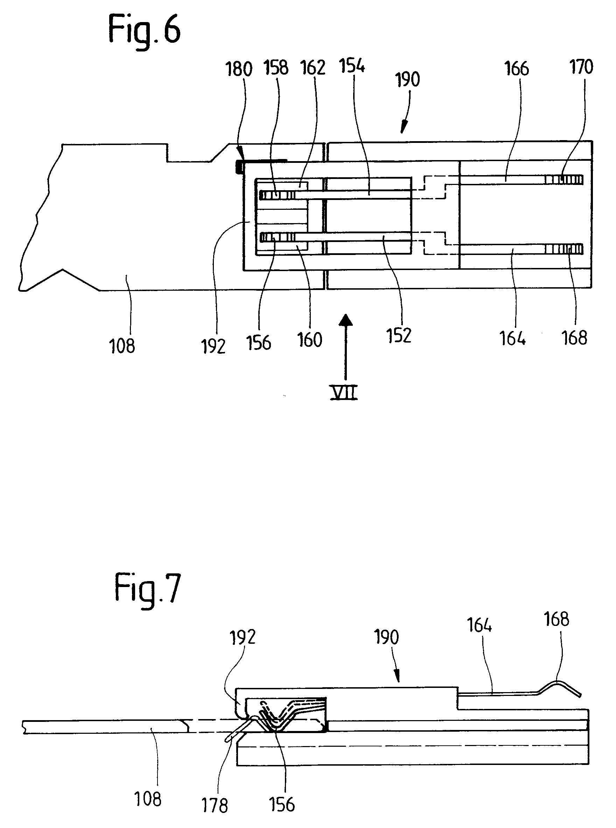

Sur les figures 6 et 7 est représentée une variante de réalisation du connecteur mobile 150 décrit ci-avant.

Sur ces figures 6 et 7, le connecteur mobile 190 diffère du connecteur mobile 150 décrit ci-avant en ce qu'il comporte en plus une structure de protection 192 servant à protéger les extrémités recourbées 156 et 158 des deux organes de contact électrique 152 et 154 lors de l'introduction d'un capteur multizone amovible 108 dans ce connecteur mobile 190. La structure de protection 192 permet ainsi d'éviter qu'une introduction répétée de capteurs multizones amovibles dans le connecteur mobile 190 endommage les extrémités recourbées 156 et 158 en les déformant, ce qui serait néfaste à l'établissement d'une connexion électrique correcte.

Dans un mode de réalisation de l'invention non représenté, il est possible de prévoir un agencement similaire à celui proposé à la figure 3, mais dans lequel au moins un des collecteurs fixes, servant à relier électriquement le connecteur mobile 150 ou 190 à un circuit de mesure, est formé par un ensemble de plages de contact électrique disposées le long du trajet prévu pour le connecteur mobile et en particulier pour l'organe de contact électrique de ce connecteur mobile associé à ce collecteur fixe.

Chacune desdites plages de contact électrique correspond à une position de mesure particulière du connecteur mobile et est agencée de manière à permettre un contact électrique avec l'organe de contact électrique associé au connecteur mobile lorsque ce dernier est situé dans cette position de mesure particulière.

On rappellera ici que les différentes positions de mesure du connecteur mobile sont déterminées par le ressort de positionnement 138, en particulier par le crochet 142 de ce ressort de positionnement 138, et par les encoches de positionnement 140 prévues le long du capteur multizone amovible 108. Les diverses plages de contact électrique mentionnées ci-avant sont reliées électriquement audit circuit électrique de mesure de manière à permettre à ce circuit électrique de mesure de déterminer dans laquelle des positions de mesure se trouve le connecteur mobile.

Dans ce dernier mode de réalisation, la connexion électrique entre le connecteur mobile et les collecteurs fixes 172 et 174 est également réalisée par deux contacts électriques glissants du type décrit précédemment.

## Revendications

1. Dispositif de mesure, destiné à être associé à un capteur multizone amovible (108), comprenant un boîtier (104), un circuit électrique de mesure agencé dans ce boîtier et un connecteur mobile (150; 190) servant à relier électriquement ce circuit électrique de mesure audit capteur multizone amovible, ce connecteur mobile comprenant des premiers moyens de contact électrique (152, 154) destinés à relier électriquement ce connecteur mobile audit capteur multizone amovible et des deuxièmes moyens de contact électrique (164, 166) reliés électriquement auxdits premiers moyens de contact électrique et servant à relier électriquement ce connecteur mobile audit circuit électrique de mesure, ce dispositif de mesure étant caractérisé en ce que lesdits deuxièmes moyens de contact électrique sont formés par au moins deux organes de contact électrique (164, 166) et en ce qu'il comprend deux collecteurs fixes (172, 174) ménagés à l'intérieur dudit boîtier de manière que lesdits deux organes de contact électrique sont respectivement en contact électrique avec ces deux collecteurs fixes lorsque ledit connecteur mobile est situé dans une quelconque position de mesure parmi une pluralité de positions de mesure différentes.

2. Dispositif de mesure selon la revendication 1, caractérisé en ce que ledit connecteur mobile (150; 190) comprend un chariot mobile (151) guidé dans une coulisse (153) ménagée dans ledit boîtier (104) de ce dispositif de mesure.

3. Dispositif de mesure selon la revendication 1 ou 2, caractérisé en ce que lesdits deux collecteurs fixes (172, 174) sont constitués respectivement par deux pistes métallisées contre lesquelles lesdits deux organes de contact électrique (164, 166) sont respectivement en appui de manière à former respectivement deux contacts électriques glissants entre lesdites deux pistes métallisées (172, 174) et ledits deux organes de contact électrique.

4. Dispositif de mesure selon la revendication 1 ou 2, caractérisé en ce qu'au moins un des deux collecteurs fixes est formé par un ensemble de plages de contact électrique disposées le long d'un trajet prévu pour ledit organe de contact électrique associé à ce collecteur fixe, chacune desdites plages de contact électrique correspondant à une position de mesure différente dudit connecteur mobile.

5. Dispositif de mesure selon l'une quelconque des revendications précédentes, caractérisé en ce que lesdits deux organes de contact électrique (164, 166) sont formés chacun par une lame élastique comprenant une extrémité libre recourbée (168, 170).

6. Dispositif de mesure selon la revendication 1, caractérisé en ce que lesdits premiers moyens de contact (152, 154) sont formés par deux lames élastiques, ces deux lames élastiques étant respectivement reliées électriquement aux deux organes de contact électrique (164, 166) et agencées de manière à être respectivement en contact électrique avec deux plages de contact électrique prévues sur ledit capteur multizone amovible (108) lorsque ce dernier est joint audit connecteur mobile (150; 190).

7. Dispositif de mesure selon l'une quelconque des revendications précédentes, caractérisé en ce que ce dispositif de mesure et ledit capteur multizone amovible (108) auquel il est associé sont agencés pour mesurer le taux de glucose dans le sang.
